(19) Europäisches Patentamt
European Patent Office
Office européen des brevets

(11) Publication number: 0 307 841
A1

(12) **EUROPEAN PATENT APPLICATION**

(21) Application number: 88114877.9

(22) Date of filing: 12.09.88

(51) Int. Cl.4: C12N 15/00 , C07H 21/04 , A01H 1/00 , A01N 65/00

The applicant has filed a statement in accordance with Rule 28 (4) EPC (issue of a sample only to an expert). Accession number(s) of the deposit(s): ATCC 67510.

(30) Priority: 15.09.87 US 96579

(43) Date of publication of application: 22.03.89 Bulletin 89/12

(84) Designated Contracting States: AT BE CH DE ES FR GB GR IT LI LU NL SE

(71) Applicant: THE GENERAL HOSPITAL CORPORATION
55 Fruit Street
Boston MA 02114(US)

(72) Inventor: Pfitzner, Ursula M.
21 Petzetstrasse
D-8000 München 60(DE)
Inventor: Pfitzner, Arthur P.
21 Petzetstrasse
D-8000 München 60(DE)
Inventor: Goodman, Howard M., Prof.
10 The Ledges Road
Newton Center, MA 02159(US)

(74) Representative: Klein, Otto, Dr. et al
Hoechst AG Zentrale Patentabteilung
Postfach 80 03 20
D-6230 Frankfurt am Main 80(DE)

# (54) Pathogenesis-related proteins in plants.

(57) This invention is directed to the regulatory sequences of the pathogenesis-related (PR) protein genes of the PR-1 group, specifically an inducible PR-1 promoter and, preferably, PR-1 secretion signal sequences, and to genetic constructs containing the regulatory sequences. This invention also relates to the construction and use of a recombinant DNA molecule that includes a PR protein gene, and its regulatory sequences, including the promoter and secretion signal sequences. More specifically, the invention concerns the use of the recombinant DNA molecule that includes a PR-protein gene operably linked to PR protein regulatory sequences that upon expression in a plant will enhance the hypersensitive response of the plant to an invading pathogen.

EP 0 307 841 A1

# PATHOGENESIS-RELATED PROTEINS IN PLANTS

## FIELD OF THE INVENTION

This invention is directed to the regulatory sequences of pathogenesis-related (PR) protein genes of the PR-1 group, specifically the inducible PR-1 protein promoters and the PR-1 secretion signal sequences, and to genetic constructs containing the regulatory sequences. This invention also relates to the construction and use of a recombinant DNA molecule that includes a PR protein gene, and its regulatory sequences, including the promoter and secretion signal sequences. More specifically, the invention concerns the use of the recombinant DNA molecule that includes a PR-protein gene operably linked to PR protein regulatory sequences that upon expression in a plant will enhance the hypersensitive response of the plant to an invading pathogen.

## BACKGROUND OF THE INVENTION

Pathogenic infection of a variety of different plant species leads to the synthesis of several host encoded proteins, known as pathogenesis-related proteins (PR proteins). van Loon, Plant Molecular Biol. 4:111-116 (1985). For example, infection of tobacco plants with tobacco mosaic virus (TMW) results in two possible distinct host responses depending on the genetic constitution of the plant cultivar and the virus strain. In a systemic infection, the virus particles are soon found spread all over the plants, resulting in the expression of several disease symptoms. By contrast, in an incompatible reaction, necrotic lesions appear on the leaves around the sites of pathogen entry and the virus particles remain restricted to these areas. This induced defense reaction, called a hypersensitive response, is common among many plant-pathogen interactions. Concomitant with the expression of disease resistance, various biochemical changes are observed in the plants. These changes include increases in the activities of the enzymes involved in the phenylpropanoid pathway and the de novo synthesis of the pathogenesis-related (PR) proteins. Matthews, R.E.F., in "Comprehensive Virology 16," (H. Fraenkel-Conrat and R.R. Wagner, eds.), Plenum Press, NY (1980), pp. 297-359.

PR-proteins were first detected in tobacco cultivars which exhibited the hypersensitive response upon TMV infection. Gianinazzi et al., C.R. Acad. Sci. Paris Ser. D270:2383-2386 (1970) and van Loon et al., Virology 40:199-211 (1970). As the appearance of these proteins was initially found to be strictly correlated with the plant defense reaction, a possible antiviral function of PR-proteins was postulated. Pierpoint, W.S., Trends in Biochem. 85:5-7 (1983). Since then, proteins with physical properties similar to PR-proteins have been described in many other plant species after infection with viruses, viroids, fungi, as well as bacteria. van Loon (1985), supra. In addition, PR-proteins can be induced artificially in healthy plants by the application of chemicals, such as acetylsalicylic acid, polyacrylic acid, and ethylene. White, R.F., Virology 99:410-412 (1979). Interestingly, these proteins can accumulate under various alternate environmental conditions: they have been in tobacco plants at the onset of flowering (Fraser, R.S.S., Physiol. Plant Pathol. 19:69-76 (1981)) and in tobacco callus cultures (Antoniw et al., Phytopath Z. 101:179-184 (1981)).

PR-proteins have several features in common. They are soluble in acidic buffers, have low molecular weights ranging from 10 kD to 20 kD, occur in the intercellular spaces of the leaves, and are resistant to proteases. van Loon (1985), supra. In TMV infected tobacco plants, ten different PR-proteins have been described. The best characterized of these is the PR-1 group which consists of three members, PR-1a, PR-1b, and PR-1c. Biochemical, serological, and genetic evidence suggest that these proteins, although closely related, are encoded by separate genes. Antoniw et al., J. Gen. Virol. 47: 79-87 (1980); Matsuoka et al., J. Gen. Virol. 65:2209-2215 (1984); Antoniw et al., Plant Mol. Biol. 4:55-60 (1985); Ahl et al., Phytopathology 72:80-85 (1982); and Gianinazzi et al., Neth. J. Plant Pathol. 89:275-281 (1983).

Lucas, et al., EMBO J. 4:2745-2749 (1985) shows the complete amino acid sequence of the PR-protein p14 from viroid-infected tomato plants and the partial sequence of the tobacco PR-protein 1a.

Cornelissen, et al., EMBO J. 5 37-40 (1986) discloses cloning pathogenesis-related proteins 1a, 1b, and 1c derived from infected tobacco plants induced by TMV. In this study, the complete nucleotide sequence of PR-1b cDNA and partial sequences of PR-1a and PR-1c was achieved.

Sommssich, et al., Proc. Nat'l Acad. Sci. USA 83:2427-2430 (1986) report cloning two cDNA parsley PR proteins, PR-1 and PR-2. In addition, by studying isolated nuclei from control and elicitor treated cells, the researchers found that PR-1 and PR-2 genes were regulated mainly at the transcriptional level. According to this study, transcription was extremely rapid and occurred within minutes after elicitation.

It would be desirable to be able to produce PR-proteins using genetic engineering techniques, and to utilize the regulatory sequences of the PR-protein gene for the expression and secretion of not only PR-proteins, but also for heterologous proteins or polypeptides.

It would also be desirable to develop plants that have an enhanced hypersensitive response to invading pathogens by increasing the levels of PR-proteins in said plants using genetic engineering techniques. In such a manner, it would be possible to confer an enhanced hypersensitive response to a plant.

## SUMMARY OF THE INVENTION

It has now been found that PR-1 promoters can be used as a promoter in recombinant DNA molecules such that a PR-1 promoter is operably linked to a heterologous gene that codes for a biologically active product. Using a PR-1 promoter to control the expression of heterologous genes has many attendant advantages. PR-1 promoter control system is tightly regulated. PR-1 promoters are chemically inducible with acetylsalioylic acid, polyacrylic acid, and ethylene. Thus, the heterologous gene is not expressed until the PR-1 promotor is induced. Upon chemical induction, the heterologous gene is transcribed as part of a messenger RNA which initiates at the PR-1 promoter. Once induced, polypeptides are produced quickly and efficiently. The inventors have further discovered that PR-1 promoters have significant homology to the heat shock promoter, but are not heat shock inducible.

Therefore, this invention is directed to the regulatory sequences of pathogenesis-related (PR) protein genes of the PR-1 group, specifically the inducible PR-1 promoters and preferably also the PR secretion signal sequences. The invention also relates to chimeric genetic constructs containing the PR-1 promoter operably linked to a heterologous gene in expression vectors and expressible in a given host. The invention is further related to chimeric genetic constructs containing both a PR-1 promoter and a PR secretion signal sequence operably linked to a heterologous gene in expression vectors and expressible in a given host.

This invention also relates to the construction and use of a recombinant DNA molecule that includes a PR-1 promoter operably linked to a pathogenesis-related (PR) protein gene, and preferably also linked to a PR-1 secretion signal sequence. The invention is also directed to methods for conferring an enhanced hypersensitive response in a plant to an invading pathogen by expressing the recombinant DNA molcule.

## BRIEF DESCRIPTION OF THE FIGURES

Figure 1 shows the gel electrophoretic analysis for protein and RNA levels in TMV infected and mock inoculated tobacco leaves.

Figure 2 shows restriction maps of PR-protein cDNA clones and DNA sequencing strategies.

Figure 3 shows the nucleotide sequences of six independent PR-protein cDNA clones.

Figure 4 gives the comparison of the amino acid sequences of PR-proteins deduced from the respective cDNA clones.

Figure 5 shows the Southern blot analysis of PR-protein genes in tobacco.

Figure 6 shows the Southern blot analysis of PR-protein genes in *Lycopersicon esculentum*.

Figure 7 gives the partial restriction maps of three independent PR-1 genomic clones.

Figure 8 shows the nucleotide sequence and deduced amino acid sequence of the PR-1a gene and 5′ and 3′ flanking regions.

Figure 9 shows the primer extension analysis of tobacco PR-1 genes.

Figure 10 gives a comparison of nucleotide sequences in the TATA proximal regions of the *Drosophila melanogaster* hsp70 promoter (Karch *et al.*, 1981) and the PR-1a gene.

## DETAILED DESCRIPTION OF THE INVENTION

In the following description, reference will be made to various methodologies well known to those of skill in the art of recombinant genetics. Publications and other materials setting forth such well known methodologies will be referred to in the course of this description, and are incorporated herein by reference in their entireties as though set forth in full. Standard reference works setting forth the general principles of recombinant DNA technology include Old, *et al.*, *Principles of Gene Manipulation*, Blackwell (1985) and Maniatis, *et al.*, *Molecular Cloning: A Laboratory Manual*, Cold Spring Harbor Laboratory Press (New York 1982).

By "promoter" is meant a DNA sequence generally described as the 5′ region of a gene, located proximal to the start codon. At the promoter region, transcription or expression of an adjacent gene is initiated. This is called the transcription initiation site.

By "inducible promoter" is meant a promoter in which the transcription of synthesis is initiated or increased by exposure of the cells to an effector.

By "heterologous gene" is meant a gene that is foreign, i.e. originating from a donor different from the host or a chemically synthesized gene and can include a donor of a different species from the host. The heterologous gene encodes a DNA sequence that may be transcribed into mRNA, which mRNA may then be translated into a sequence of amino acids characteristic of a specific peptide. Typically the first transcribed nucleotide is numbered +1, and the nucleotides are numbered consecutively with positive integers throughout the translated region of the structural gene and into the 3′ untranslated region. The numbering of nucleotides in the promoter and regulatory region 5′ to the transcribed region proceeds consecutively with negative integers. Thus the 5′ nucleotide of the promoter and regulatory region adjacent to the first transcribed nucleotide of the structural gene would be numbered -1.

By "operably linked" is meant that the promoter controls the initiation of expression of the polypeptide encoded by the structural gene.

## A. Pathogenesis-Related Protein Regulatory Sequences: Inducible Promoter and Secretion Signal

The present invention provides for the PR-1 protein regulatory sequences, specifically coding for inducible PR-1 protein promoters and, preferably also PR secretion signal sequences. As previously stated, the PR-1 group consists of three closely related members: PR-1a, PR-1b, and PR-1c. Evidence indicates that there is approximately 90% homology in the amino acid structure of the proteins. As used herein, PR-1 is meant to include all three members of the group: PR-1a, PR-1b, and PR-1c.

There is thus provided in this invention a promoter with the following nucleotide sequence:

```
-335                          -305                -285
            .           .           .           .           .
GATCGTTAAATGTAGAAAATATTAATTAACACATTAACCATAACCAGTCT
            -275                    -255                -235
            .           .           .           .           .
ACTTTATTTAACAAAAAGCACATCTGATAGATCAAAAAAGTGTTTAACTT
            -225                    -205                -185
            .           .           .           .           .
CATGCATTGACAATTTAAAATTATTTTGCAACATCGGGTAAAACTATTTT
            -175                    -155                -135
            .           .           .           .           .
ACAACAATTGGTAACTGCATATATAAGTTTAATATGGTAACCTAGAAAAT
            -125                    -105                 -85
            .           .           .           .           .
AGGATAAATTATCTATAACAGGATATATTACATTGATATTACCATGTCAA
            -75                     -55                  -35
            .           .           .           .           .
AAAATTTAGTAAGTACATGAATAATCACCGTGAAATCTTCAAGATTTCTC
            -25                      -5   +1
            .           .           .     *
CTATAAATACCCTTGGTAGTAAATCTAGTTTTTCCA
```

The inventors have discovered that the inducible PR-1 protein promoters have significant homology with the consensus heat shock element (HSE) promoter. The region of dyad symmetry (-54 to -38) located 3 bp upstream of the TATA box (-34 to -27), contains part of a perfect match to the consensus HSE (-57 to -43), the only difference being the insertion of one additional nucleotide. The sequence C--GAA---TTC--G, which differs from the consenus heat shock regulatory element only by the insertion of one extra nucleotide, is located at position -57. The presence of this element suggests a molecular relationship of heat shock and pathogen responses in plants. However, according to studies conducted by the inventors as reported in Ohashi and Matsuoka, Plant Cell Physiol., 26:473-480 (1985), Pr-1 genes are not induced to detectable levels under heat shock conditios. In addition, it has been demonstrated that PR-protein synthesis is completely suppressed after shifting tobacco plants from 20 degrees C to 30 degrees C (van Loon, Physiol. Plant Pathol. 6:289-300 (1975).

The use of a PR-1 protein promoter in genetic constructs according to this invention is advantageous because it can be induced through the use of chemicals, such as acetylsalicylic acid, polyacrylic acid, or ethylene. Thus, the inducible PR-1 protein promoter can be used to regulate the expression of heterologous genes. In one embodiment of this invention, a PR-1 protein promoter is operably linked to a genetic sequence coding for a desired heterologous protein, preferably a PR protein gene. The resulting genetic construct is introduced into or forms part of an expression vehicle. The expression vehicle is then utilized to transform an appropriate host. The host is cultured or grown under conditions selected to achieve optimum growth. A PR-1 protein promoter will not express the heterologous gene until it is chemically treated, which induces the promoter to initiate expression of the heterologous gene. The sequence of actions include transcription of the gene into mRNA, and the translation thereof into the protein.

In yet another aspect of this invention, the PR-1 regulatory sequence also contains a secretion signal sequence which may be used to regulate the secretion of an expressed heterologous protein or polypeptide. In plants, the pathogenesis-related proteins are secreted into the intercellular spaces of the plant cell. When expressing a heterologous gene using genetic engineering techniques, the secretion of a recombinant protein into the culture media is often desirable for ease of recovering and purifying the protein. Accordingly, in one aspect of this invention, PR-1 secretion signal sequence may be used to regulate the secretion of heterologous proteins. The amino acid sequence of a PR secretion signal is shown in Figure 4 and comprises amino acids beginning with -30 to -1.

Thus, in another embodiment of this invention, the secretion signal is linked to a promoter, which may or may not be the PR-promoter, which together are operably linked in the correct reading frame to a gene coding for the desired heterologous protein. The resulting genetic construct is introduced or made part of an expression vehicle which transforms an appropriate host cell. After proper culturing conditions, the gene is expressed, with the added improvement that the expressed protein or polypeptide is secreted into the culture medium. The secreted protein or polypeptide can easily be isolated and purified according to known means.

The PR-protein gene and the PR-1 regulatory sequences of the PR promoter and secretion signal have been isolated from tobacco plants. However, the practice of this invention is not limited to this source for the PR-1a regulatory sequences. Current evidence suggests that all plants have to some degree the pathogenesis-related proteins. The PR-1 regulatory sequences may be isolated from these plants.

Further, it will be understood in the art that there may be some differences in the nucleotide sequence between various plant species, due to the degeneracy of the genetic code, environmental factors and evolution. However, minor changes may be made in the PR-1 protein regulatory sequences described herein, such as shortening or lengthening the sequences, fusing various fragments of the PR-1 protein regulatory sequences or making nucleotide substitutions where one or more nucleotides have been deleted or substituted by other nucleotides without departing from the scope of this invention. The only requirement for the PR-1 promoter is that the substitutions, deletions, additions, or fragments be functional for expressing a heterologous gene.

In addition, the PR gene and promoter and secretion signal may be synthesized by manipulation in the laboratory, rather than of natural origin. Thus, for example, even though a given complete sequence for a naturally-occurring PR-1 protein promoter and secretion signal may exist integrated into the genomic DNA of a given plant species, the isolated sequence corresponding to a PR-1 protein promoter separated from the genomic DNA of the plant, with or without adjacent sequences corresponding to the structural gene, start or stop signals, is not naturally occurring and would be considered to be "synthetic." In addition, given the degeneracy of the genetic code, the knowledge of an amino acid sequence does not necessarily and irrevocably lead to the naturally occurring genetic sequence coding therefore.

A PR-1 promoter according to this invention may be operably linked to a genetic sequence coding for any heterologous protein that may be expressed in host cells. Examples of such proteins include, but ar not limited to, enzymes, hormones, hemoglobin, interferons, interleukins, and growth factors. The use of a PR-1 promoter will be described relating to various embodiments of the invention comprising pathogenesis-related proteins, although it is to be understood that a PR-1 promoter may be operably linked to a genetic seqence coding for any protein or polypeptide that may be expressed according to recombinant DNA techniques.

## B. Expression of the Pathogenesis-Related Protein in Plants

In another embodiment of this invention, a PR-protein gene is used to transform a plant to enhance the hypersensitive response of the plant to an invading pathogen.

The coding region for a PR-protein gene that may be used in this invention may be homologous or heterologous to the plant cell or plant being transformed. It is necessary, however, that the genetic sequence coding for a PR-protein be expressed, and produced, as a functional protein or polypeptide in the resulting plant cell. Thus, the invention comprises plants containing either homologous PR-protein genes or heterologous PR-protein genes that express the PR-protein. Further, the PR-protein gene may be from other plant species.

In one embodiment of this invention, a PR-protein comprises a plant PR-protein that is homologous to the plant to be transformed. In another embodiment of this invention, a PR-protein comprises a plant PR-protein that is heterologous to the plant to be transformed. The preferred PR-protein is the PR-1a protein described herein in the Examples. However, other PR-genes are known and may be used in this invention. Moreover, DNA from both genomic DNA and cDNA encoding a PR-protein gene may be used in this invention. Further, a PR-protein gene may be constructed partially of a cDNA clone and partially of a genomic clone. In addition, the DNA coding for the PR-protein gene may comprise portions from various plants.

There are a variety of embodiments encompassed in the broad concept of the invention. In one of its embodiments, this invention comprises chimeric genetic sequences:

(a) a first genetic sequence coding for a PR-protein that upon expression of the gene in a given plant cell is functional for PR-protein;

(b) one or more additional genetic sequences operably linked on either side of the PR-protein coding region. These additional genetic sequences contain sequences for promoter(s) or terminator(s). The plant regulatory sequences may be heterologous or homologous to the host cell.

In a preferred embodiment, the promotor of a PR-1 protein gene is used to express the chimeric genetic sequence. Other promoters that may be used in the genetic sequence include nos, ocs, and CaMV promoters. An efficient plant promoter that may be used is an overproducing plant promoter. This promoter in operable linkage with the genetic sequence for PR-protein should be capable of promoting expression of said PR-protein such that the transformed plant is tolerant or capable of resisting an invading pathogen. Overproducing plant promoters that may be used in this invention include the promoter of the small subunit (ss) of the ribulos-1,5-biphosphate carboxylase from soybean (Berry-Lowe et al., J. Molecular and App. Gen., 1:483-498 (1982), and the promoter of the chlorophyll a/b binding protein. These two promoters are known to be light induced in eucaryotic plant cells (see, for example, Genetic Engineering of Plants, an Agricultural Perspective, A. Cashmore, Plenum, New York 1983, pages 29-38; Corruzi, G. et al., J. of Biol. Chem., 258: 1399 (1983); and Dunsmuir, P. et al., J. of Mol. and Applied Genet., 2: 285 (1983)).

Further, in another preferred embodiment, the expression of the chimeric genetic sequence comprising the PR-protein gene is operably linked in correct reading frame with a plant promoter and with the PR-1a gene secretion signal sequence.

The chimeric genetic sequence comprising a PR-protein gene operably linked to a plant promoter, and in the preferred embodiment with the secretion signal sequences, can be ligated into a suitable cloning vector. In general, plasmid or viral (bacteriophage) vectors containing replication and control sequences derived from species compatible with the host cell are used. The cloning vector will typically carry a replication origin, as well as specific genes that are capable of providing phenotypic selection markers in transformed host cells, typically resistance to antibiotics. The transforming vectors can be selected by these phenotypic markers after transformation in a host cell.

Host cells that may be used in this invention include procaryotes, including bacterial hosts such as E. coli, S. typhimurium, and Serratia marcescens. Eucaryotic hosts such as yeast or filamentous fungi may also be used in this invention.

The cloning vector and host cell transformed with the vector are used in this invention typically to increase the copy number of the vector. With an increased copy number, the vectors containing the PR-protein gene can be isolated and, for example, used to introduce the chimeric genetic sequences into the plant cells. The genetic material contained in the vector can be microinjected directly into plant cells by use of micropipettes to mechanically transfer the recombinant DNA. The genetic material may also be transferred into the plant cell by using polyethylene glycol which forms a precipitation complex with the genetic material that is taken up by the cell. (Paszkowski et al., EMBO J. 3:2717-22 (1984)).

In an alternative embodiment of this invention, the PR-protein gene may be introduced into the plant cells by electroporation. (Fromm et al., "Expression of Genes Transferred into Monocot and Dicot Plant Cells by Electroporation," Proc. Nat'l. Acad. Sci. U.S.A. 82:5824 (1985)). In this technique, plant protoplasts are electroporated in the presence of plasmids containing the PR-protein genetic construct. Electrical impulses of high field strength reversibly permeabilize biomembranes allowing the introduction of the plasmids. Electroporated plant protoplasts reform the cell wall, divide, and form plant callus. Selection of the transformed plant cells with the expressed PR-protein can be accomplished using the phenotypic markers as described above.

Another method of introducing the PR-protein gene into plant cells is to infect a plant cell with Agrobacterium tumefaciens transformed with the PR-protein gene. Under appropriate conditions known in the art, the transformed plant cells are grown to form shoots, roots, and develop further into plants. The PR-protein genetic sequences can be introduced into appropriate plant cells, for example, by means of the Ti plasmid of Agrobacterium tumefaciens. The Ti plasmid is transmitted to plant cells on infection by Agrobacterium tumefaciens and is stably integrated into the plant genome. Horsch et al., "Inheritance of Functional Foreign Genes in Plants," Science 233:496-498 (1984); Fraley et al., Proc. Nat'l Acad. Sci. U.S.A. 80:4803 (1983)).

Ti plasmids contain two regions essential for the production of transformed cells. One of these, named transfer DNA (T DNA), induces tumor formation. The other, termed virulent region, is essential for the formation but not maintenance of tumors. The transfer DNA region, which transfers to the plant genome, can be increased in size by the insertion of the PR-protein genetic sequence without its transferring ability being affected. By removing the tumor-causing genes so that they no longer interfere, the modified Ti plasmid can then be used as a vector for the transfer of the gene constructs of the invention into an appropriate plant cell.

All plant cells which can be transformed by Agrobacterium and whole plants regenerated from the transformed cells can also be transformed according to the invention so to produce transformed whole plants which contain the transferred PR-protein gene.

There are presently two different ways to transform plant cells with Agrobacterium:

(1) co-cultivation of Agrobacterium with cultured isolated protoplasts, or

(2) transforming cells or tissues with Agrobacterium.

Method (1) requires an established culture system that allows culturing protoplasts and plant regeneration from cultured protoplasts.

Method (2) requires (a) that the plant cells or tissues can be transformed by Agrobacterium and (b) that the transformed cells or tissues can be induced to regenerate into whole plants. In the binary system, to have infection, two plasmids are needed: a T-DNA containing plasmid and a vir plasmid.

After transformation of the plant cell or plant, those plant cells or plants transformed by the Ti plasmid so that the PR-protein is expressed, can be selected by an appropriate phenotypic marker. These phenotypical markers include, but are not limited to, antibiotic resistance. Other phenotypic markers are known in the art and may be used in this invention.

All plants from which protoplasts can be isolated and cultured to give whole regenerated plants can be transformed by the present invention so that whole plants are recovered which contain the transferred PR-protien gene. Some suitable plants include, for example, species from the genera Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum, Sorghum, and Datura.

There is an increasing body of evidence that practically all plants can be regenerated from cultured cells or tissues, including but not limited to all major cereal crop species, sugarcane, sugar beet, cotton, fruit and other trees, legumes and vegetables. Limited knowledge presently exists on whether all of these plants can be transformed by Agrobacterium. Species which are a natural plant host for Agrobacterium may be transformable in vitro. Monocotyledonous plants, and in particular, cereals and grasses, are not natural hosts to Agrobacterium. Attempts to transform them using Agrobacterium have been unsuccessful until recently. Hooykas-Van Slogteren et al., Nature 311:763-764 (1984). There is growing evidence now that certain monocots can be transformed by Agrobacterium. Using novel experimental approaches that have now become available, cereal and grass species may be transformable.

Additional plant genera that may be transformed by Agrobacterium include Ipomoea, Passiflora, Cyclamen, Malus, Prunus, Rosa, Rubus, Populus, Santalum, Allium, Lilium, Narcissus, Ananas, Arachis, Phaseolus, and Pisum.

Plant regeneration from cultural protoplasts is described in Evans et al., "Protoplast Isolation and Culture," in Handbook of Plant Cell Culture 1:124-176 (MacMillan Publishing Co., New York, 1983); M.R. Davey, "Recent Developments in the Culture and Regeneration of Plant Protoplasts," Protoplasts, 1983 - Lecture Proceedings, pp. 19-29 (Birkhauser, Basel, 1983); P. J. Dale, "Protoplast Culture and Plant Regeneration of Cereals and Other Recalcitrant Crops," in Protoplasts 1983-Lecture Proceedings, pp. 31-41 (Birkhauser, Basel, 1983); and H. Binding, "Regeneration of Plants," in Plant Protoplasts, pp. 21-37 (CRC Press, Boca Raton, 1985).

Regeneration varies from species to species of plants, but generally a suspension of transformed protoplasts containing multiple copies of the PR-protein gene is first provided. Embryo formation can then be induced from the protoplast suspensions, to the stage of ripening and germination as natural embryos. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

The mature plants, grown from the transformed plant cells, are selfed to produce an inbred plant. The inbred plant produces seed containing the gene for the increased PR-protein. These seeds can be grown to produce plants that have enhanced hypersensitive response of the plant to an invading organism.

The inbreds according to this invention can be used to develop hypersensitive responsive hybrids. In this method a hypersensitive responsive inbred line is crossed with another hypersensitive responsive inbred line to produce the hybrid.

Parts obtained from the regenerated plant, such as flowers, seeds, leaves, branches, fruit, and the like are covered by the invention provided that these parts comprise the hypersensitive responsive cells. Progeny and variants, and mutants of the regenerated plants are also included within the scope of this invention.

In diploid plants, typically one parent may be transformed by the PR-protein genetic sequence and the other parent is the wild type. After crossing the parents, the first generation hybrids (F1) will show a distribution of 1/2 PR/WT (wild type):1/2 PR/WT. These first generation hybrids (F1) are selfed to produce second generation hybrids (F2). The genetic distribution of the F2 hybrids are 1/4 PR/PR:1/2 PR/WT (wild type):1/4 WT/WT. The F2 hybrids with the genetic makeup of PR/PR are chosen as the hypersensitive responsive plants.

As used herein, variant describes phenotypic changes that are stable and heritable, including heritable variation that is sexually transmitted to progeny of plants, provided that the variant still comprises a hypersensitive responsive plant. Also, as used herein, mutant describes variation as a result of environmental conditions, such as radiation, or as a result of genetic variation in which a trait is trans mitted meiotically according to well-established laws of inheritance. The mutant plant, however, must still exhibit a hypersensitive response as according to the invention.

PNtW38gPR1a (containing the PR-1a promoter, structural gene, and 3′ end) in E. coli was deposited at the American type Culture Collection (ATCC), 12301 Parklawn Drive, Rockville, Maryland 20852 USA under ATCC accession no. 67510 on September 9, 1987.

Having now generally described this invention, the same will be better understood by reference to specific examples, which are included herein for purposes of illustration only, and are not intended to be limiting unless otherwise specified.

## EXAMPLE 1

### MATERIALS AND METHODS

#### Plant Material

Plants (Nicotiana tabacum cv. Samsun NN and Lycopersicon esculentum) were grown under normal greenhouse conditions at 20° C. Two to three months old tobacco plants were infected by rubbing a suspension of purified TMV ((3); common strain, 30 µg/ml) onto the leaves and the plants transferred to a growth chamber. Control plants were obtained by mock inoculation of leaves with 0.1 M sodium phosphate buffer, pH 7. Leaves were harvested 7 days after the inoculation procedure, frozen in liquid nitrogen, and stored at -70° C.

#### RNA and DNA isolation

Total cellular RNA was isolated from the frozen leaves by the guanidium/cesium chloride method (4) except that the extraction buffer was 4 M guanidium isothiocyanate, 50 mM Tris-HCl pH 7.6, 10 mM EDTA, 2% sodium lauryl sarkosinate, and 0.1% β-mercaptoethanol. Poly $(A)^+$ RNA was selected by oligo(dT)-cellulose chromatography (5). Total DNA was extracted from three months old tobacco plants and six months old tomato plants according to the method in reference (6). Plasmid DNA was isolated by the alkaline lysis method (7).

Construction and screening of the cDNA library

Double stranded cDNA was synthesized from 5 µg poly(A)$^+$ RNA isolated from TMV infected Samsun NN leaves (8). After homopolymeric tailing with dC-residues, the cDNA was annealed to PstI cut, dG-tailed pUC12 and transformed into E. coli strain DK1. Plating and screening of the transformants was as described (9). A 30 nucleotide long synthetic oligodeoxynucleotide, 5′-GTCTTGTTGAGAGTTTTGGGCACGACAAGA-3′, complementary to PR-protein mRNA (10) was end-labeled with [gamma-$^{32}$P]ATP by T4 polynucleotide kinase (specific activity $8 \times 10^8$ cpm/µg) and used as a probe. Hybridizations were performed at 42°C in 6xSSC (1xSSC = 0.15 M NaCl, 0.015 M sodium citrate, pH 7.0), 10xDenhardt's solution (1xDenhardt's = 0.2 g/l each of Ficoll, polyvinylpyrrolidone, and bovine serium albumin), 50mM HEPES pH 7.0, and 162.5 µg/ml denatured salmon sperm DNA. Filters were washed at room temperature, at 42°C, and at 60°C in 6xSSC for 3 x 15 minutes each.

DNA sequence analysis

Nucleotide sequences were determined using the dideoxynucleotide chain termination method (11) with (α-$^{35}$S]dATP (12) and the universal external M13 primer. Restriction enzyme fragments were subcloned into M13mp18 and mp19 vectors. In order to sequence the 3′-terminus of pNt$^{SNN}$cPR1a/8 on both DNA strands, a synthetic 15 base long oligodeoxynucleotide (5′-TCAAGTAGCTAGACC-3′) complementary to the very end of the clone was used as primer.

Genomic Southern blots

Ten µg of plant DNA were digested to completion with BamHI or HindIII and separated by electrophoresis on 0.8% agarose gels. The DNA was transferred onto nitrocellulose filters (4) and hybridized with nick translated pNt$^{SNN}$cPR1-a/35 probe (specific activity $5 \times 10^8$ cpm/µg). In order to evaluate nonspecific hybridization with vector sequences, parallel blots were hybridized with nick translated pUC12 probe. Hyridizations were performed according to the method in reference (4). All filters were washed for 15 minutes at room temperature in 2xSSC, 0.5 %SDS. Tobacco DNA blots were further washed for 1 hr at 68°C in 0.3xSSC, 0.5% SDS. Filters with tomato DNA were wahed for 1 hr at 68°C in either 1xSSC, 0.5% SDS or 0.5xSSC, 0.5% SDS. Copy number calculations are based on a haploid genome size of 3.9 pg for Nicotiana tabacum.

Northern blots

Thirty µg of total cellular RNA were denatured in glyoxal (13), and separated by electrophoresis on 1.5% agarose gels. The RNAs were transferred onto nitrocellulose filters, probed with the 30 base synthetic oligodeoxynucleotide, hybridized, and washed using the conditions described in "Construction and screening of the cDNA library" above. The positions in the gel of the 28S and 18S rRNA bands were used as standards for size estimation of the hyridizing band.

Antibodies directed against PR-proteins

PR-proteins were extracted from frozen leaves with two volumes of citrate phosphate buffer (32 mM $Na_2HPO_4$, 84 mM citric acid, pH 2.8) containing 0.1% β-mercaptoethanol. The crude protein extract was concentrated by the addition of ammonium sulfate (95% final concentration) and separated by chromatofocusing. PR-proteins were eluted from the column at pH 3.9 (PR-1b) and pH 3.5 (PR-1a). These fractions were further purified by electrophoresis on 11.25% native polyacrylamide gels prepared as described in reference (14). For immunizations, two New Zealand White Rabbits were each injected with an emulsion of crushed polyacrylamide gel slices containing 30 µg PR-1a or PR-1b in complete Freund's adjuvant. Booster injections were administered 2 weeks after the initial injections, and the rabbits were bled 1 week and 2 weeks later. The antisera were used without further purification. Complete immunological identity of PR-1a and PR-1b was shown by the Ouchterlony double diffusion method.

Immunoblots

Crude protein extracts were concentrated by the addition of acetone (80% final concentration). Precipitated proteins were redissolved in SDS gel loading buffer, subjected to electrophoresis on 15% polyacrylamide gels (15), and blotted onto nitrocellulose membranes (16). The filters were incubated in a 1:5 dilution of the specific antiserum directed against PR-1a. Immunodetection was achieved by Protein-A coupled horseradish peroxidase according to the instructions of the manufacturer (Bio-Rad). The positions in the gel of protein standards (Pharmacia, LMW calibration kit proteins) were used for size estimation of PR-proteins.

RESULTS

Induction of PR-proteins in TMV infected tobacco plants

Equal amounts of protein isolated from TMV infected and mock inoculated tobacco plants (*Nicotiana tabacum* cv. Samsun NN) were separated on SDS-polyacrylamide gels and blotted onto nitrocellulose membranes. Figure 1 shows the results of this test.

In Figure 1, the following data are depicted:

A: Samples of 40 µg of protein isolated from TMV infected (lane 1) or mock inoculated (lane 2) leaves were analyzed for PR-protein content by SDS polyacrylamide gel electrophoresis followed by immunoblotting. The positions in the gel of a standard set of protein size markers are shown.

B: Samples of 30 µg of total cellular RNA isolated from TMV infected (lane 1) or mock inoculated (lane 2) leaves were denatured in glyoxal, separated on a 1.5% agarose gel, blotted onto a nitrocellulose filter, and hybridized with an end-labeled 30 base long oligodeoxynucleotide complementary to PR-protein mRNA. The positions in the gel of the 28S and 18S rRNA bands were used as standards for size estimation of the hybridizing band. The nature of the counts at the origin of the gel is unknown.

When these filters were probed with a PR-1a specific polyclonal antiserum, a single 18kD protein band was seen with extracts from the virus infected leaves (Fig. 1A, lane 1), but not with mock inoculated control plants (Fig. 1A, lane 2). The same plants were also used as a source for RNA extraction. When an RNA blot with equal amounts of total cellular RNA per lane was hyridized with a 30 nucleotide long synthetic oligodeoxynucleotide probe complementary to PR-protein mRNA (10) no hybridization was detected with RNA isolated from control plants (Fig. 1B, lane 2). With RNA from TMV infected leaves, however, a single transcript of approximately 700 nucleotides is readily detectable (Fig. 1B, lane 1). The size of this RNA is sufficient to encode a protein of 18 kD.

Isolation and characterization of tobacco PR-protein cDNA clones

Starting with 5 µg poly(A)$^+$ RNA isolated from TMV infected tobacco leaves, a cDNA library of 30,000 recombinant clones was constructed in the plasmid vector pUC12 (8). This library was screened for the presence of PR-protein sequences by hydridization with the 30 nucleotide long synthetic probe. Out of 15,000 transformants, a total of 176 PR-protein specific clones were identified. This corresponds to a level of PR-protein mRNAs of ~1%, assuming an equal conversion of each mRNA species into cDNA. Subsequently, 32 clones were purified and characterized. Thirty clones varied in insert size from 700 bp to 800 bp suggesting that they might represent nearly complete copies of their respective mRNAs, a view supported by the fact that the synthetic probe is complementary to sequences encoding amino acids at the N-terminus of PR-proteins.

By digestion with different restriction endonucleases, three classes of PR-protein cDNA clones were identified. Figure 2 shows restriction maps of PR-protein cDNA clones and DNA sequencing strategies. The restriction maps show only those restriction endonuclease sites relevant in distinguishing the three classes of PR-protein cDNA clones and the conserved PstI site in the protein coding regions. Thick black bars, protein coding regions of mature proteins; thick open bars, protein coding regions of signal peptides; thin bars, 3′-untranslated regions. Horizontal arrows under each clone indicate direction and extent of sequence determinations. All clones share a conserved PstI site in the protein coding regions (Fig. 2), but differ in the unique restriction sites in their 3′-untranslated regions. Class I clones (20 members) are characterized by an EcoRV site, class II clones (6 members) by SspI and EcoRI sites, and class III clones (4 members) by NdeI and HhaI sites (Fig. 2). Members of each group, the two longest clones of their class, were further characterized by DNA sequence analysis.

Only minor differences in the DNA sequences were found between cDNA clones of the same class. Figure 3 shows nucleotide sequences of six independent PR-protein cDNA clones. The strands homologous to the respective mRNAs are shown. Coding sequences are in upper case letters and 3′-untranslated regions in lower case letters. Only the sequence of the longest clone of each class is given in its entirety and nucleotides are numbered in the 5′ to 3′ direction. Differences in sequence of the shorter clone of each class are shown in bold; the numbering of the longest clone is retained irrespective of insertions or deletions. Putative polyadenylation signals are underlined. PR1a: The sequence of pNt$^{SNN}$cPR1a/8 is shown starting at nucleotide (nt) #14. The 30 nucleotide long probe is complementary to (nt) #74 to 103 with two mismatches at nt #79 and 91. The sequence of the other PR-1a clone, pNt$^{SNN}$cPR1a/35 (shown in bold on the line under pNt$^{SNN}$cPR1a/8), was identical to that shown except that its 5′ terminus was at position #1 (i.e. it has 13 additional nucleotides at its 5′ end, nt #1-13 shown in bold), it has an A (rather than a G) at nt #361, it has an A residue added at nt #680 shown by a →+a), and has a 77 nt shorter 3′ untranslated region. The poly(dA) is attached to a C at nt #695 in pNt$^{SNN}$cPR1a/35 (seven A's; $dA_7$) and to a G at nt #772 in pNt$^{SNN}$cPR1a/8 (48 A's; $dA_{48}$). Pr1b: The sequence of pNt$^{SNN}$cPR1b/81 is shown. The 30 nucleotide long probe is complementary to nucleotides (nt) #69 to 98 with two mismatches at nt #73 and 76. The sequence of the other PR-1b clone, pNt$^{SNN}$cPR1b/1, was identical to that shown except that its 5′ terminus was at position #1 (i.e. it has 13 additonal nucleotides at its 5′ end, nt #1-13 shown in bold) and it has a 50 nt shrter 3′ untranslated region. Neither clone has a poly(dA) tract. PR1c: The sequence of pNt$^{SNN}$cPR1c/49 is shown. The 30 nucleotide long probe is complementary to nucleotides (nt) #60 to 89 with two mismatches at nt #67 and 71. The sequence of the other PR-1c clone, pNt$^{SNN}$cPR1c/83, was identical to that shown except that it has a 58 nt shorter 3′ untranslated region. The poly(dA) is attached to an A at nt #663 in pNt$^{SNN}$cPR1c/83 (35 A's; $dA_{29}$) and no poly(dA) tract is apparent in pNt$^{SNN}$cPR1c/49. The sequence of both strands was determined for the entire clone of pNt$^{SNN}$cPR1c/49 (class III), greater than 60% for pNt$^{SNN}$cPR1b/81 (class II) and greater than 80% for pNt$^{SNN}$cPR1a/8 (class I). For the three other clones the sequences were only determined on one strand.

The clones are therefore derived from the same gene or from allelic genes. The clone with the longest open reading frame (499 nucleotides) was pNt$^{SNN}$cPR1a/35. Two clones, pNt$^{SNN}$cPR1a/8 and pNt$^{SNN}$cPR1c/83, had long poly(dA) tails with 48 and 35 residues, respectively, while another clone, pNt$^{SNN}$cPR1a/35, has only seven dA-residues. Assuming these represent the in vivo polyadenylation sites, the PR-protein cDNA clones differ in the lengths of their 3′-untranslated regions; 194 nucleotides for pNt$^{SNN}$cPR1a/35 and 270 nucleotides for pNt$^{SNN}$cPR1a/8 (both class I) and 169 nucleotides for pNt$^{SNN}$cPR1c/83 and >233 nucleotides for pNt$^{SNN}$cPR1c/49 (both class III). The two class II clones analyzed (pNt$^{SNN}$cPR1b/1 and /81) terminated prior to the poly(dA) tail. The PR-protein genes therefore use alternative polyadenylation sites, a finding in agreement with observations made for other plant genes (17). Furthermore, the highly conserved polyadenylation signal AATAAA, which is located 10-30 bp upstream of the poly(dA) addition site in most mammalian genes (18) is not present in the PR-protein cDNA clones. However, the related sequences ATAATT and AAAAAT (pNt$^{SNN}$cPR1a/35), AATCAT (pNt$^{SNN}$cPR1a/8), and ATAATA (pNt$^{SNN}$cPR1c/83) occur at the correct spacing with respect to the positions of the poly(dA) tracts and therefore might function as polyadenylation signals (17). The overall homology of PR-protein cDNA clones at the nucleotide level is 90% in the protein coding regions and 80% in the 3′-untranslated regions.

## Amino acid sequences of tobacco PR-proteins

Figure 4 is a comparison of the amino acid sequences of PR-proteins deduced from the respective cDNA clones. The PR-1a protein sequence as deduced from the nucleotide sequence of pNt$^{SNN}$cPR1a/35 is shown. Only amino acids which are different from PR-1a are shown for PR-1b and PR-1c as deduced from the nucleotide sequences of pNt$^{SNN}$cPR1b/1 and pNt$^{SNN}$cPR1c/83, respectively. Identical amino acids are indicated by blanks, differences by the changed amino acid, and conservative changes (V→I, R→H, and A→V) are indicated by (*). Residues are numbered beginning with 1 for the first amino acid of the mature proteins (Q, shown in bold). Negative numbers indicate amino acids of the signal peptides. The processing site is between the A at -1 and the Q at +1. Every tenth residue in each direction has a dot over it. The single letter amino acid code is used.

Comparison of the amino acid sequences of the PR-proteins deduced from the cDNA clones, indicates that the sequences are identical for the same clone class and very similar between classes (Fig. 4). The assignment of the cDNA clones to the respective proteins was based on comparison with the amino acid sequence of protein PR-1a (1) and nucleotide sequences of PR-1a, PR-1b and PR-1c (2). Class I clones correspond to PR-1a and represent the mRNA expressed at the highest level, class II clones correspond to PR-1b, and class III cDNA clones, which are found at the lowest level, correspond to PR-1c. The abundance level of the mRNA was assumed to be directly related to the number of cDNA clones isolated of each type. Four amino acid differences are observed, when the predicted amino acid sequence for PR-1a is compared to data obtained by Edman degradation of purified PR-1a (1). These are Asp→Ser at position 27, Ser→Pro at position 38, Gln→Ser at position 39, and Tyr→Trp at position 132.

The longest amino acid sequence (166 residues) was obtained for PR-1a, however, it is still missing the initiation methionine at position -30 (known to occur at this position from sequence data on a lambda clone from a genomic tobacco library with essentially an identical sequence with this cDNA (A.J.P. Pfitzner, U.M. Pfitzner, and H.M. Goodman, unpublished results). For PR-1b and PR-1c, amino acid sequences of 164 and 161 residues, respectively, were obtained. Both of these sequences contain a methionine in the correct reading frame at position -22. However, it is not likely to be the start of translation since another in frame methionine located eight residues upstream has been previously described for PR-1b and PR-1c (2) and the sequences surrounding Met -22 in PR-1b and PR-1c do not agree with the favored nucleotide sequences flanking functional initiation codons of eukaryotic mRNAs (19). Clone $pNt^{SNN}cPR1a/35$ (PR-1a) is therefore presumably missing five nucleotides, $pNt^{SNN}cPR1b/1$ (PR-1b) ten nucleotides, and $pNt^{SNN}cPR1c/49$ (PR-1c) nineteen nucleotides of their signal peptide coding regions.

PR-proteins are synthesized as precursor molecules with N-terminal extensions (10, 10). The cleavage site (indicated in Fig. 4) of the signal peptides is predicted from the amino acid sequence established for the mature protein PR-1a (1) and the pre-protein sequence as deduced from the nucleotide sequence. PR-protein signal peptides share common features with other eukaryotic signal sequences (21). These include a hydrophilic N-terminal region, a hydrophobic core, and a hydrophilic stretch of six amino acids at the C-terminus. The amino acids in the positions -1 and -3 relative to the processing site are polar and/or small (Ala, Cys, Ser), whereas the residues in the positions -2 and +1 are charged amino acids and/or have bulky side chains (His, Arg, Gln).

The mature PR-proteins each contain 138 amino acids. This corresponds to a calculated molecular weight of 15,225 for PR-1a, 15,075 for PR-1b, and 15-129 for PR-1c and is in reasonable agreement with the $M_r$ of 18 kD estimated by SDS polyacrylamide gel electrophoresis. The homology of PR-proteins at the amino acid level is 90% since some nucleotide changes are silent. Variations in the amino acid sequences are predominantly found in the signal peptide and at the C-terminus.

## Identification of PR-protein genes in tobacco and tomato plants

The organization of PR-protein genes was investigated by Southern blot analysis as shown in Figure 5 which shows Southern blot anaylsis of PR-protein genes in Nicotiana tabacum cv. Samsun NN. Ten µg of total DNA were digested to completion with endonucleases BamHI (lane 1) or HindIII (lane 2). The digests were separated on a 0.8% agarose gel, and blotted onto a nitrocellulose filter. The filter was hybridized with nick translated $pNt^{SNN}cPR1a/35$ probe. Hybridization due to nonspecific binding to vector sequences alone is marked by (x). Lanes 3 and 4 show hybridization signals from linearized $pNt^{SNN}cPR1a/35$ corresponding to 10 gene copies and 1 gene copy, respectively. The positions in the gel of a HindIII digest of wild type bacteriophage lambda DNA were used as standards for size estimation of the hybridizing bands.

Total DNA from Nicotiana tabacum cv. Samsun NN digested with endonucleases BamHI or HindIII and probed with $pNt^{SNN}cPR1a/35$ detects seven genomic fragments ranging in size from 18 kb to 7 kb (BamHI) or 16 kb to 2 kb (HindIII) (Fig. 5). When the three different PR cDNAs were used to probe parallel blots only minor differences in relative band intensities were observed. Since neither BamHI nor HindIII are known to restrict PR-protein cDNA clones this result suggests that the PR-protein genes in tobacco are organized in a small multigene family consisting of approximately seven members. In order to evaluate the generality of this observation, DNA from tomato plants was isolated and hybridized with a tobacco cDNA probe. One major PR-protein from tomato plants (1), p14, which has about 60% homology with the tobacco proteins at the amino acid level and is markedly induced after viroid infection has been described. On Southern blots, however, at least three genomic fragments hybridize to the tobacco probe even at high stringency as shown in Figure 6. Figure 6 shows Southern blot analysis of PR-protein genes in Lycopersicon esculentum. Tenµg of total DNA isolated from tobacco plants (lane 1) or tomato plants (lane 2) were digested to completion with endonuclease BamHI. The DNA transfer blots were washed either in 1xSSC, 0.5% SDS (panel A.) or in 0.5xSSC, 0.5% SDS (panel B.). Thus, the genomes of tobacco as well as tomato plants contain several PR-1 related genes.

## EXAMPLE 2

### Materials and Methods

#### Plant Material

Tobacco plants were grown under normal greenhouse conditions at 20° C. When 2 to 3 months old, the plants were mock-inoculated or infected with tobacco mosaíc virus as described (25) and harvested 7 days later.

#### RNA and DNA Isolation

RNA was extracted from tobacco leaves as described (25). Poly(A)$^+$ RNA was selected by oligo(dT)-cellulose chromatography (5). Plasmid DNA was isolated by the alkaline lysis method (7).

#### Screening of the Genomic Library

About $10^6$ recombinant plaques of a tobacco (Nicotiana tabacum cv. Wisconsin 38) genomic library established in the backteriophage lambda vector EMBL3 provided by Mark A. Conkling were screened using nick-translated pNt$^{SNN}$cPR1a/35 probe (specific activity 5 x $10^8$ cpm/µg, (25)). Filter hybridizations were performed according to standard procedures (4) and positive plaques were purified twice. DNA of recombinant clones was prepared from liquid cultures grown on the E. coli K12 strain LE392 and analyzed by digestion with restriction enzymes.

#### DNA Sequence Analysis

Nucleotide sequences were determined using the dideoxynucleotide chain termination method (11) with [$\alpha$-$^{35}$S]dATP (12) and the universal external M13 primer. Restriction enzyme fragments were subcloned into M13mp18 and mp19 vectors (26) prior to sequencing.

#### Primer Extension Analysis

A synthetic 30 bp oligdeoxynucleotide complementary to positions 108 to 137 in the sequence (Figure 2) was end-labeled with T4 polynucleotide kinase and [gamma-$^{32}$P]ATP (specific activity 8 x $10^8$ cpm/µg). About 7.5 ng primer were annealed to 0.5 µg poly(A)$^+$ RNA from either mock-inoculated or TMV-infected tobacco plants in a 15 µl reaction containing 100 mM Tris-HCl, pH 8.3, 10 mM $MgCl_2$, 140 mM KCl, 20 mM $\beta$-mercaptoethanol, 1 mM dNTPs, and 40 units RNAsin (Promega Biotech). The reaction was carried out at 42° C for 45 minutes in the presence of 21 units AMV reverse transcriptase (Life Sciences). The primer extension products were analyzed on a 6% polyacrylamide-8M urea sequencing gel along with the products of a dideoxy sequencing reaction generated by using the same primer and an M13mp18 template containing the 0.6 kb SalI/PstI fragment of the PR-1a gene.

### Results

Isolation and Characterization of Genomic Clones Encoding PR-1 Genes

A tobacco (Nicotiana tabacum cv. Wisconsin 38) genomic library constructed in the bacteriophage lambda vector EMBL3 was screened by plaque hybridization using the cDNA clone $pNt^{SNN}cPR1a/35$ (25). Out of $10^6$ recombinant phages, 4 PR-1-specific clones were obtained. Analysis of these clones by digestion with various restriction endonucleases and hybridization of the resulting fragments with the cDNA probe revealed that the clones are derived from three different genomic loci.

Figure 7 shows the partial restriction maps of three independedent PR-1 genomic clones. The approximate locations of the PR-1 genes within the clones as determined by hybridization with a PR-1a cDNA probe is shown by the solid boxes and the relative orientation by the arrows. Abbreviations used: B, BamHI; E, EcoRI; H, HindIII; K, KpnI; P, PstI; S, SalI.

Each clone contains only one single gene copy. The approximate locations of the PR-1 genes are confined to a 2.2 kb HindIII fragment in lambda W38/1, to a 2 kb EcoRI fragment in lambda W38/3, and to a 1.4 kb SalI/EcoRI fragment in lambda W38/5 (Fig. 7). These fragments were subcloned into pUC18 or pUC19 vectors. For further analysis, the clones were digested with restriction endonucleases specific for the 3′ untranslated regions of cDNA clones encoding PR-1a, PR-1b, and PR-1c proteins, respectively, and hybridized with the 340 bp PstI/EcoRV fragment of $pNt^{SNN}cPR1a/35$. One of the genomic clones, lambda W38/5, gave rise to a PstI/EcoRV fragment identical in size to the corresponding fragment obtained from the cDNA clone $pNt^{SNN}cPR1a/35$ (data not shown). This result therefore suggested that lambda W38/5 encodes the PR-1a protein and that intervening sequences are not present in this region of the gene.

Primary Structure of the PR-1a Gene

The genomic nucleotide sequence of the 1.4 kb SalI/EcoRI fragment of lambda W38/5 is shown in Figure 8.

Figure 8 shows the nucleotide sequence and deduced amino acid sequence of the PR-1a gene and 5′ and 3′ flanking regions. The sequence of the coding strand is shown numbered in the left-hand column and by a dot above every tenth nucleotide. The nucleotide sequence of the genomic clone is compared to that of two cDNA clones published previously (25). Identical nucleotides are indicated by blanks, differences by the changed nucleotide and amino acid substitutions are given next to the changed nucleotide in parentheses. The cDNA sequence starts at position 35 marked by the vertical arrow. Two in vivo polyadenylation sites at positions 732 and 808 are indicated. The major start of transscription, A, is labeled +1. Important features described in the text are marked as follows: The consensus TATA box at position -34 and an inverted match to the CCAAT sequence at position -179 are shown in bold letters. Repeat elements are overlined and their relative orientation is given by horizontal arrows. The HSE-like motif at position -57 is indicated. Matches to the consensus HSE are denoted by (*) and mismatches by (-).

The DNA sequence of the gene is identical to the previously isolated cDNA clones $pNt^{SNN}cPR1a/8$ and $pNt^{SNN}cPR1a/35$ encoding the PR-1a protein from Nicotiana tabacum cv. Samsun NN apart from 3 nucleotide changes. Two of these substitutions are located in the coding region and give rise to amino acid changes. These are G→A (Asp106→Asn106) at position 345, and T→A (Ser153→Thr153) at position 486. The third change, T→C, is located at position 601 in the 3′ untranslated region of the gene. Several lines of evidence indicate that these differences represent slight variations in the primary structures of PR-1a proteins from different tobacco cultivars:

(1) The other two genomic clones, lambda W38/1 and lambda W38/3, are clearly different in their DNA sequences from cDNA clone previously characterized, and are obviously derived from pseudogenes.

(2) One of the amino acid changes, Ser153→Thr153, is a conservative one.

(3) The other amino acid substitution, Asp106→Asn106, is observed at the same position in the cDNA clones encoding the PR-1c protein (25).

(4) Genomic Southern blots with DNA from Samsun NN and Wisconsin 38 tobacco yield nearly identical hybridization patterns suggesting that both cultivars contain an equal number of highly homologous PR-1 genes (data not shown).

(5) Treatment of Samsun NN as well as Wisconsin 38 tobacco with acetylsalicylic acid leads to the induction of PR-1 proteins displaying similar electrophoretic mobilities on two-dimensional polyacrylamide gels (data not shown). Thus, clone lambda W38/5 most presumably contains an active gene encoding the PR-1a protein from Nicotiana tabacum cv. Wisconsin 38.

The genomic sequence contains an uninterrupted open reading frame of 504 nucleotides. The primary structure of the PR-1a protein is homologous to the PR-1b and PR-1c proteins including a 30-amino acid signal peptide typical of eukaryotic exoproteins and a mature protein of 138 amino acids in length (2, 25). PR-1 genes do not contain introns. At the 3′ end of the PR-1a gene, multiple polyadenylation sites have previously been identified resulting in 194 bp and 270 bp long 3′ noncoding regions (25). The DNA site corresponding to the 5′ end of PR-1 mRNAs was determined by primer extension experiments. Only one major extension product was observed using poly(A)$^+$ RNA from TMV infected leaves.

Figure 9 shows the primer extension analysis of tobacco PR-1 genes. A synthetic oligodeoxynucleotide homologous to PR-1 genes was used to prime synthesis on 0.5 µg poly(A)$^+$ RNA from mock inoculated tobacco plants (lane 1) and from TMV infected plants (lane 2). The arrow indicates the major mRNA reverse transcription product observed (lane 2). The sequencing ladder (lanes GATC) was generated with the same oligodeoxynucleotide primer and an M13mp18 template containing the 0.6 kb SalI/PstI fragment of the PR-1a gene. The sequence of the noncoding strand shown in the figure is indicated next to the gel. Only the relevant region of the gel is shown; no other bands were seen elsewhere in the gel.

The position of the transcription initiation site defined by this experiment is identical to the 5′ ends of cDNA clones encoding the PR-1b and PR-1c proteins characterized previously (2). Thus, the 5′ untranslated leader regions are presumably of an equal length being 29 nucleotides for all PR-1 genes.

## 5′ Nucleotide sequence of the PR-1a gene

The 335 bp genomic 5′ flanking sequence of the PR-1a gene contained in clone lambda W38/5 was determined by DNA sequence analysis. Visual inspection of this region led to the identification of structural elements, which might be involved in the regulation of PR-1 gene expression by environmental stimuli. At position -34 with respect to the start of transcription (+1, Fig. 8), a consensus TATA box and at position -179 an inverted match to the CCAAT sequence were found (22). Furthermore, an imperfect direct repeat of 11 bp is present at positions -116 and -135, and an 8 bp inverted repeat is located at positions -45 and -54 (Figure 8).

When this region was searched for the presence of transcriptional signals known to occur in other eukaryotic genes, a striking homology to the TATA proximal sequences of the 70 kd Drosophila melanogaster heat shock protein (hsp70) promoter was apparent.

Figure 10 shows the comparison of nucleotide sequences in the TATA proximal regions of the Drosophila melanogaster hsp70 promoter (23) and the PR-1a gene. Gaps were introduced in the PR-1a sequence in order to maximize homology. Sequences matching the consensus for TATA (black box) and the heat shock regulatory element (HSE) (stippled box) are shown in bold letters. Horizontal arrows mark inverted repeats. Numbering is relative to the respective transcription start sites (in bold face) designated +1 and the direction of transcription is indicated.

At position -57, the sequence C--GAA---TTC--G, is found which differs from the consensus heat shock regulatory element (HSE) only by the insertion of one extra nucleotide in the middle of the motif (24). In addition, another imperfect homology to the HSE (6 out of 8 nucleotides) is located at position -70 overlapping the downstream HSE-like motif (Figure 8).

Although the foregoing refers to particular preferred embodiments, it will be understood that the present invention is not so limited. It will occur to those ordinarily skilled in the art that various modifications may be made to the disclosed embodiments and that such modifications are intended to be within the scope of the present invention.

The references cited herein are grouped in the following bibliography and respectively cited parenthetically by number in the foregoing text are hereby incorporated by reference.

## References


1. Lucas et al., EMBO J. 4:2745-2749 (1985).
2. Cornelissen et al., EMBO J. 5:37-40 (986).
3. Leberman, R., Virology 30:341-347 (1966).
4. Maniatis et al., "Molecular cloning: a laboratory manual." Cold Spring Harbor Laboratory, Cold Spring Harbor, New York (1982).
5. Aviv, H., and Leder, P., Proc. Natl. Acad. Sci. USA 69:264-268 (1982).
6. Murray, M.G., and Thompson, W.F., Nucl. Acids Res. 8:4321-4325 (1980).
7. Birnboim, H.C., and Doly, J., Nucl. Acids Res. 7:1513-1523 (1979).

8. Gubler, U., and Hoffman, B.J., Gene 25:263-269 (1983).
9. Hanahan, D., and Meselson, M., Gene 10:63-67 (1980).
10. Hooft van Huijsduijnen et al., EMBO J. 4:2167-2171 (1985).
11. Sanger et al., Proc. Natl. Acad. Sci. USA 74:5463-5467 (1977).
12. Biggin et al., Proc. Natl. Acad. Sci. USA 80:3963-3965 (1983).
13. McMaster, G.K., and Carmichael, G.G., Proc. Natl. Acad. Sci. USA 74:4835-4838 (1977).
14. Davis, B.J., Ann. N.Y. Acad. Sci. 121:404-427 (1964).
15. Laemmli, U.K., Nature 227:680-685 (1970).
16. Burnette, W.N., Anal. Biochem. 112:195-203 (1981).
17. Dean et al., Nucl. Acids Res. 14:2229-2240 (1986).
18. Proudfoot, N.J., and Brownlee, G.G., Nature 263:211-214 (1976).
19. Kozak, M., Nucl. Acids Res. 12:857-872 (1984).
20. Carr et al., Proc. Natl. Acad. Sci. USA 82:7999-8003 (1985).
21. von Heijne, G., J. Mol. Biol. 184:99-105 (1985).
22. Breathnach, U., and Chambon, P., Ann. Rev. Biochem. 45:349-384 (1981).
23. Karch et al., J. Mol. Biol. 148:219-230 (1981).
24. Pelham, H.R.B., Trends Genet. 1:31-35 (1985).
25. Pfitzner, U.M., and Goodman, H.M., Nucl. Acids Res. 15:4449-4465 (1987).
26. Yanisch-Perron et al., Gene 33:103-119 (1985).

## Claims

1. A genetic sequence expressible in a host cell comprising the sequence encoding a PR-1 promoter sequence having the following nucleotide sequence:

```
-335                                    -305                   -285
             .             .             .             .             .
GATCGTTAAATGTAGAAAATATTAATTAACACATTAACCATAACCAGTCT
           -275                         -255                   -235
             .             .             .             .             .
ACTTTATTTAACAAAAAGCACATCTGATAGATCAAAAAAGTGTTTAACTT
           -225                         -205                   -185
             .             .             .             .             .
CATGCATTGACAATTTAAAATTATTTTGCAACATCGGGTAAAACTATTTT
           -175                         -155                   -135
             .             .             .             .             .
ACAACAATTGGTAACTGCATATATAAGTTTAATATGGTAACCTAGAAAAT
           -125                         -105                    -85
             .             .             .             .             .
AGGATAAATTATCTATAACAGGATATATTACATTGATATTACCATGTCAA
           -75                          -55                     -35
             .             .             .             .             .
AAAATTTAGTAAGTACATGAATAATCACCGTGAAATCTTCAAGATTTCTC
           -25                           -5     +1
             .             .             .      *
CTATAAATACCCTTGGTAGTAAATCTAGTTTTTCC
```

operably linked in correct reading frame to a heterologous gene.

2. The genetic sequence of claim 1 further comprising a sequence encoding a PR-1 secretion signal which together with said PR-1 promoter are in correct reading frame with heterologous gene.

3. The genetic sequence of claim 1 or 2 wherein said heterologous gene is a pathogenesis-related protein gene.

4. A genetic sequence expressible in a host cell comprising the sequence encoding a PR-1 promoter having the following nucleotide sequence:

```
-335                                     -305                   -285
           .             .             .             .             .
GATCGTTAAATGTAGAAAATATTAATTAACACATTAACCATAACCAGTCT
        -275                             -255                   -235
           .             .             .             .             .
ACTTTATTTAACAAAAAGCACATCTGATAGATCAAAAAAGTGTTTAACTT
        -225                             -205                   -185
           .             .             .             .             .
CATGCATTGACAATTTAAAATTATTTTGCAACATCGGGTAAAACTATTTT
        -175                             -155                   -135
           .             .             .             .             .
ACAACAATTGGTAACTGCATATATAAGTTTAATATGGTAACCTAGAAAAT
        -125                             -105                    -85
           .             .             .             .             .
AGGATAAATTATCTATAACAGGATATATTACATTGATATTACCATGTCAA
         -75                              -55                    -35
           .             .             .             .             .
AAAATTTAGTAAGTACATGAATAATCACCGTGAAATCTTCAAGATTTCTC
         -25                               -5   +1
           .             .             .    *
CTATAAATACCCTTGGTAGTAAATCTAGTTTTTCC
```

operably linked to a sequence encoding a PR-1 secretion signal in correct reading frame with a pathogenesis-related protein gene.

5. A plant comprising cells transformed by the genetic sequence of claim 4.

6. Plants and the seed thereof regenerated from the plant cell of claim 5.

7. Progeny of plants regenerated from the plant cell of claim 6, said progeny including mutants and variant progeny.

8. A method of producing a plant with an enhanced hypersensitive response to invading pathogens comprising transforming a plant with the genetic sequence of claim 4.

9. A recombinant DNA molecule comprising an inducible PR-1 promoter or functional fragments thereof.

10. The PR-1 promoter of claim 9, wherein said promoter is induced by chemical means.

11. The PR-1 promoter of claim 10, wherein said promoter is induced by acetylsalicylic acid, polyacrylic acid or ethylene.

Claims for the following Contracting State: ES:

1. A process for expressing a heterologous gene in a host cell, characterized by operably linking said heterologous gene in correct reading frame to the PR-1 promoter having the following nucleotide sequence

```
-335                                  -305                 -285
            .            .            .            .            .
GATCGTTAAATGTAGAAAATATTAATTAACACATTAACCATAACCAGTCT
           -275                       -255                 -235
            .            .            .            .            .
ACTTTATTTAACAAAAAGCACATCTGATAGATCAAAAAAGTGTTTAACTT
           -225                       -205                 -185
            .            .            .            .            .
CATGCATTGACAATTTAAAATTATTTTGCAACATCGGGTAAAACTATTTT
           -175                       -155                 -135
            .            .            .            .            .
ACAACAATTGGTAACTGCATATATAAGTTTAATATGGTAACCTAGAAAAT
           -125                       -105                  -85
            .            .            .            .            .
AGGATAAATTATCTATAACAGGATATATTACATTGATATTACCATGTCAA
           -75                        -55                   -35
            .            .            .            .            .
AAAATTTAGTAAGTACATGAATAATCACCGTGAAATCTTCAAGATTTCTC
           -25                         -5    +1
            .            .            .      *
CTATAAATACCCTTGGTAGTAAATCTAGTTTTTCC
```

or a functional fragment thereof.

2. A process according to claim 1, characterized in that a sequence encoding a PR-1 secretion signal together with said PR-1 promoter are in correct reading frame with the said heterologous gene.

3. A process according to claim 1 or 2, characterized in that said heterologous gene is a pathogenesis-related protein gene.

4. A process according to one or more of the preceding claims, characterized in that the promoter is inducible by chemical means.

5. A process according to claim 4, characterized in that said promoter is induced by acetylsalicylic acid, polyacrylic acid or ethylene.

6. A process according to one or more of the preceding claims, characterized in that the host cell is a plant cell.

7. A process for producing a plant and the seeds thereof, characterized by regenerating a cell obtained according to claim 6.

8. A method of producing a plant with an enhanced hypersensitive response to invading pathogens, characterized by transforming a plant with the genetic sequence obtained by the process of claim 3.

Neu eingereicht / Newly filed
Nouvellement déposé
(R 35)



FIG. 1

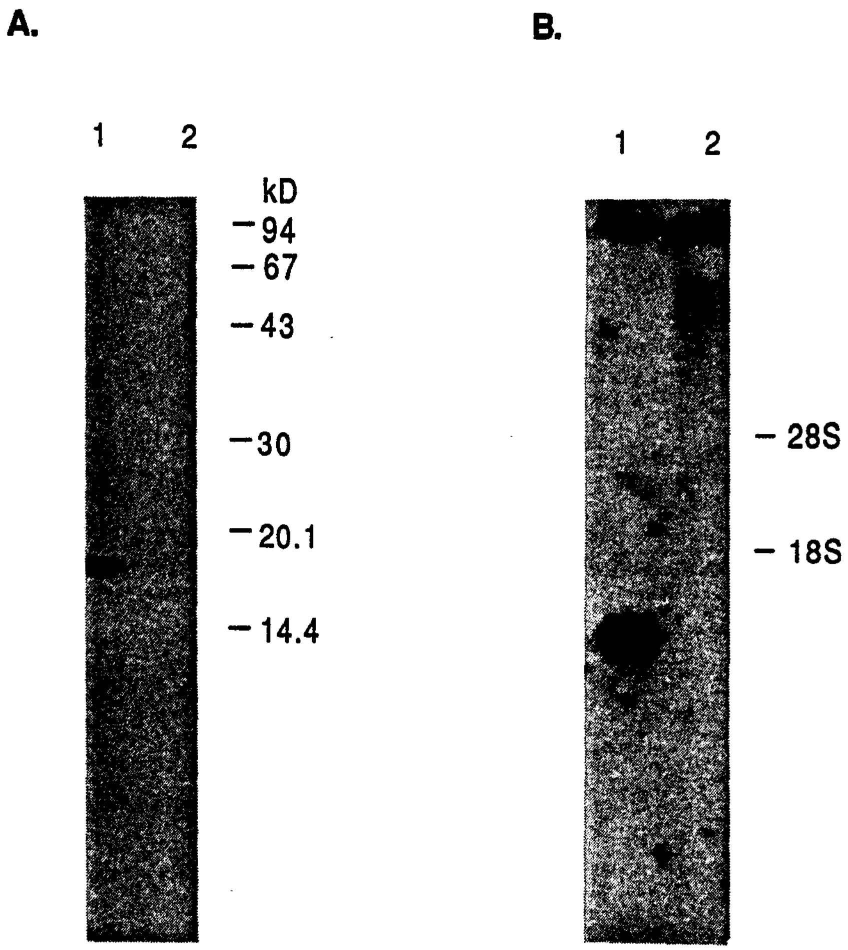

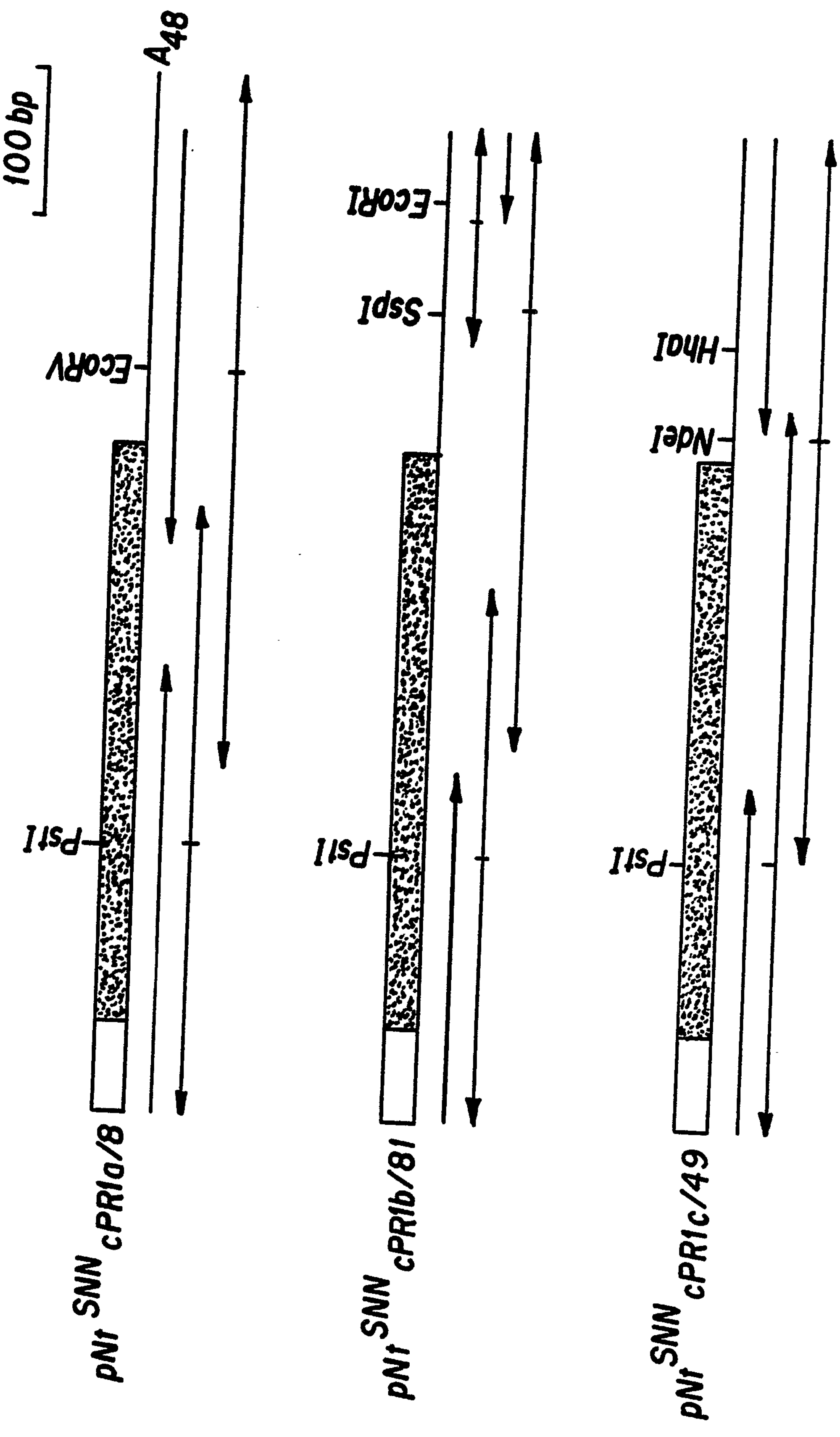

100 bp
A48
EcoRV
PstI
EcoRI
SspI
HhaI
NdeI
pNt SNN cPR1a/8
pNt SNN cPR1b/81
pNt SNN cPR1c/49


FIG. 2

pNt$^{SNN}$cPR1a/8

1 ATTTGTTCTC TTTTCACAAT TGCCTTCATT TCTTCTTGTC TCTACACTTC TCTTATTCCT

61 AGTAATATCC CACTCTTGCC GTGCCCAAAA TTCTCAACAA GACTATTTGG ATGCCCATAA

121 CACAGCTCGT GCAGATGTAG GTGTAGAACC TTTGACCTGG GACGACCAGG TAGCAGCCTA

181 TGCGCAAAAT TATGCTTCCC AATTGGCTGC AGATTGTAAC CTCGTACATT CTCATGGTCA

241 ATACGGCGAA AACCTAGCTG AGGGAAGTGG CGATTTCATG ACGGCTGCTA AGGCCGTTGA

301 GATGTGGGTC GATGAGAAAC AGTATTATGA CCATGACTCA AATACTTGTG CACAAGGACA

361 GGTGTGTGGA CACTATACTC AGGTGGTTTG GCGTAACTCG GTTCGTGTTG GATGTGCTAG

A

421 GGTTCAGTGT AACAATGGAG GATATGTTGT CTCTTGCAAC TATGATCCTC CAGGTAATTA

481 TAGAGGCGAA AGTCCATACT AAttgaaacg acctacgtcc atttcacgtt aatatgtatg

541 gattgttctg cttgatatca agaacttaaa taattgctct aaaaagcaac ttaaagtcaa

601 gtatatagta atagtactat atttgtaatc ctctgaagtg gatctataaa aagaccaagt

661 ggtcataatt aaggggaaaa tatgagttga tgatcagctt gatgtatgat ctgatattat

↑+o ↑$dA_7$

721 tatgaacact tttgtactca tacgaatcat gtgttgatgg tctagctact tg-$dA_{48}$

pNt$^{SNN}$cPR1b/81

1 TTCTCTTTTC ACAAATGCCC TCATTTTTTC TTGTCTCTAC ACTTCTCTTA TTCCTAATAA

61 TATCTCACTC TTCTCATGCC CAAAACTCTC AACAAGACTA TTTGGATGCC CATAACACAG

121 CTCGTGCAGA TGTAGGCGTG GAACCATTAA CTTGGGACAA CGGGGTAGCA GCCTATGCAC

181 AAAATTATGT TTCTCAATTG GCTGCAGACT GCAACCTCGT ACATTCTCAT GGCCAATACG

241 GCGAAAACCT AGCTCAGGGA AGTGGCGATT TTATGACGGC TGCTAAGGCC GTCGAGATGT

301 GGGTCGATGA GAAACAGTAC TATGACCATG ACTCAAATAC TTGTGCACAA GGACAGGTGT

361 GTGGACACTA TACTCAGGTG GTTTGGCGTA ACTCGGTTCG TGTTGGATGT GCTAGGGTTA

421 AGTGCAACAA TGGAGGATAT GTTGTCTCTT GCAACTATGA TCCTCCAGGT AATGTCATAG

481 GCCAAAGTCC ATACTAAttg aaatgaatgt ccatttcacg ttatatatgt atggacttct

541 gcttgatata tataaacaac ttaaataatt gcactaaaaa gcaacttata gttaaaagta

601 tataatattt gtaatcctct gaagaactgg atctgtaaaa agtccaagtg gtcttaatta

661 agggggggag gatatatgaa ttcagcttga tgtatgatct gatattatta tgaactcttt

↑ end

721 agtactctta cggaa

**FIG. 3**

pNt$^{SNN}$cPR1c/49

1 CACAAATGTC TTCATTTTTT CTTGTCTCTA CGCTTCTCTT ATTCCTAATA ATATCCCACT

61 CTTGTCATGC TCAAAACTCT CAACAAGACT ATTTGGATGC CCATAACACA GCTCGTGCAG

121 ATGTAGGTGT AGAACCTTTG ACCTGGGACG ACCAGGTAGC AGCCTATGCA CAAAATTATG

181 CTTCCCAATT GGCTGCAGAT TGTAACCTCG TACATTCTCA TGGTCAATAC GGCGAAAACC

241 TAGCTTGGGG AAGTGGCGAT TTCTTGACGG CCGCTAAGGC CGTCGAGATG TGGGTCAATG

301 AGAAACAGTA TTATGCCCAC GACTCAAACA CTTGTGCCCA AGGACAGGTG TGTGGACACT

361 ATACTCAGGT GGTTTGGCGT AACTCGGTTC GTGTTGGATG TGCTAGGGTT CAGTGTAACA

421 ATGGAGGATA TATTGTCTCT TGCAACTATG ATCCTCCAGG TAATGTTATA GGCAAAAGCC

481 CATACTAAtt gaaaacatat gtccatttca cgttatatat gtgtggactt ctgcttgata

541 tatatcaaga acttaaataa ttgcgctaaa aagcaactta tagttaagta tatagtacta

601 tatttgtaat tctctgaagt ggatatataa taagacctag tgctcttgat tatggggaaa

661 aaatatgaat tccgcttgat gtatgatatg atattattat gaaatcttta gtacttttac

721 g ↑$dA_{29}$

*FIG. 3 (cont.)*

```
                      .         .        -1         .         .         .
PR-1a    -30  MGFVLFSQLPSFLLVSTLLLFLVISHSCRAQNSQQDYLDAHNTARADVGVEPLTWDDQVA
                                    *     *

PR-1b    -30   G F    MP  F         I    SH                           NG

PR-1c    -30   E V    MS  F         I    CH                           DQ

                       .         .         .         .         .         .
PR-1a     31  AYAQNYASQLAADCNLVHSHGQYGENLAEGSGDFMTAAKAVEMWVDEKQYYDHDSNTCAQ
                    *
PR-1b     31        V                     Q     M          D     D
                    *
PR-1c     31        A                     W     L          N     A

                       .         .         .         .
PR-1a     91  GQVCGHYTQVVWRNSVRVGCARVQCNNGGYVVSCNYDPPGNYRGESPY
PR-1b     91                         K      V          VI Q
                                            *
PR-1c     91                         Q      I          VI K
```

FIG. 4

FIG. 5

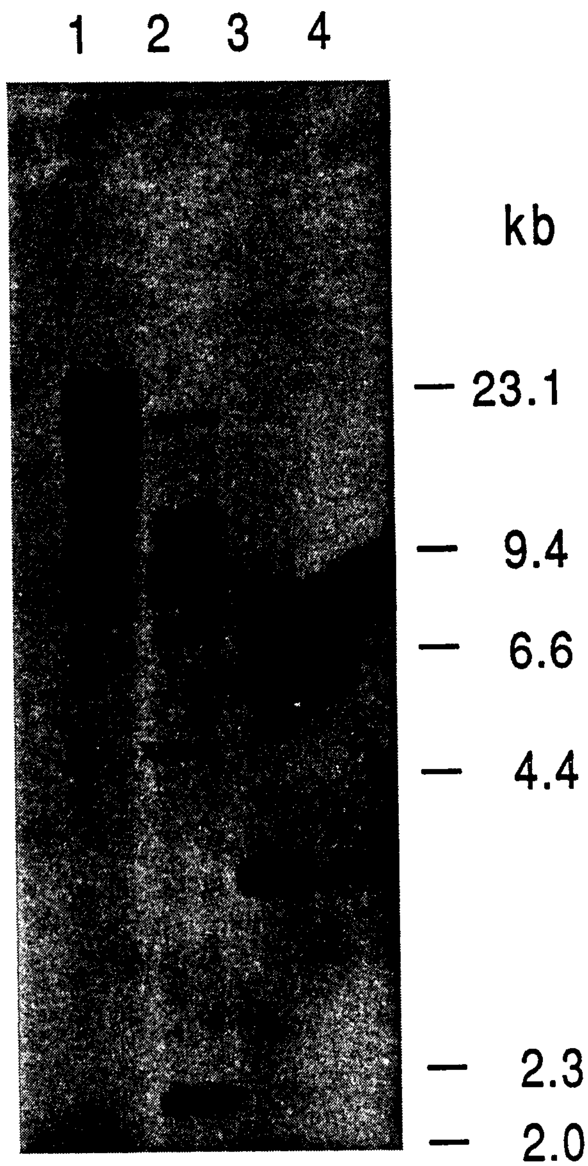

FIG. 6

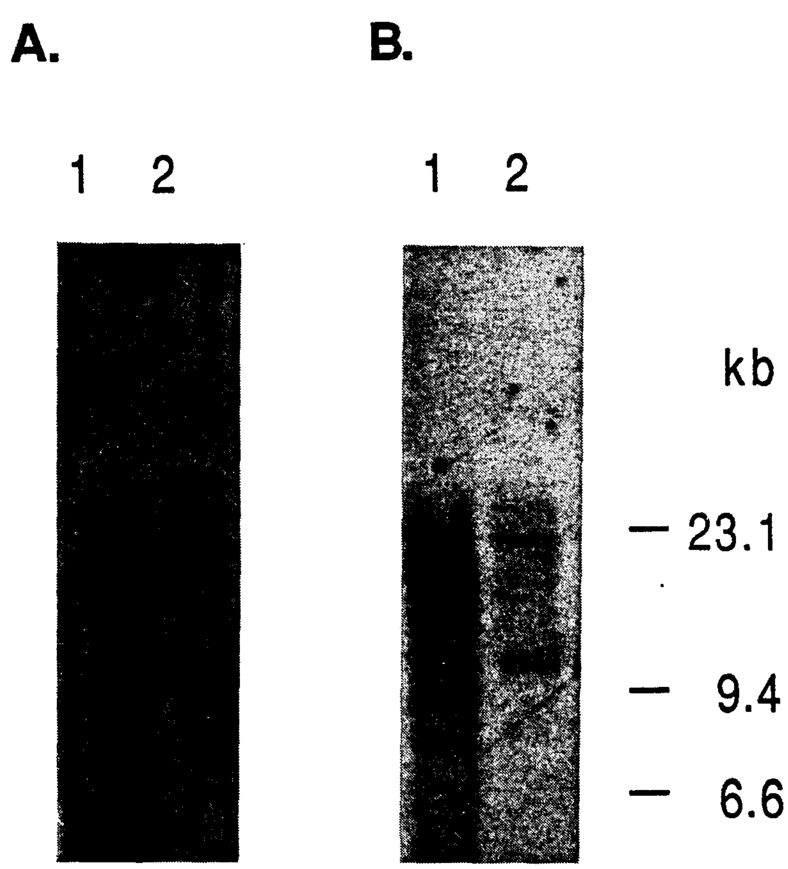

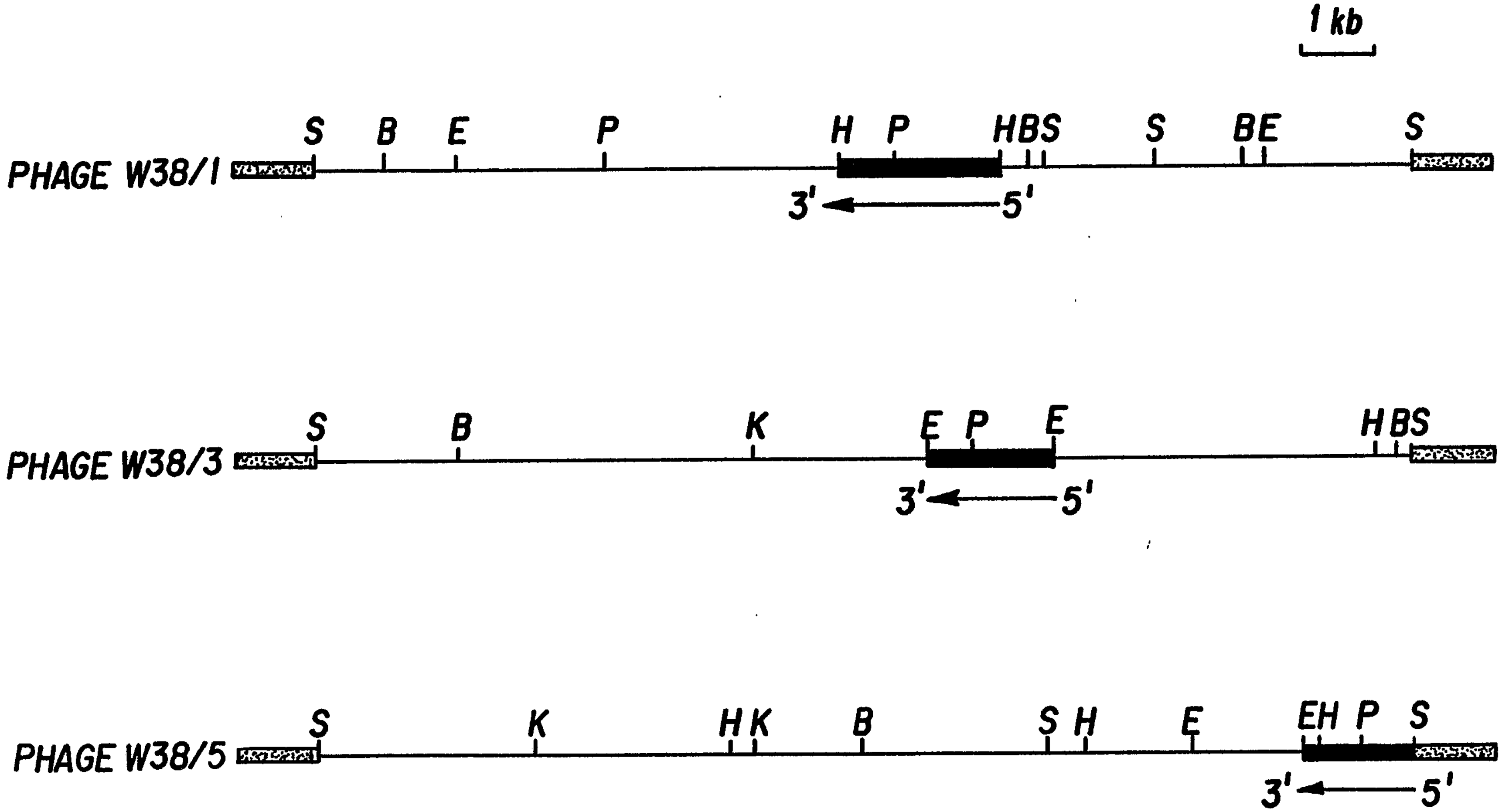
1 kb
PHAGE W38/1
S B E P H P HBS S BE S
3' 5'
PHAGE W38/3
S B K E P E HBS
3' 5'
PHAGE W38/5
S K H K B S H E EH P S
3' 5'

FIG.7

-335 GATCGTTAAATGTAGAAAATATTAATTAACACATTAACCATAACCAGTCTACTTTATTTAACAAAAAGCACATCTGATAG

-255 ATCAAAAAAGTGTTTAACTTCATGCATTGACAATTTAAAATTATTTTGCAACATCGGGTAAAACTATTTTACAACAATTG

-175 GTAACTGCATATATAAGTTTAATATGGTAACCTAGAAAATAGGATAAATTATCTATAACAGGATATATTACATTGATATT

H S E

-95 ACCATGTCAAAAAATTTAGTAAGTACATGAATAATCACCGTGAAATCTTCAAGATTTCTCCTATAAATACCCTTGGTAGT

+1 HSE-like motif

-15 AAATCTAGTTTTTCCATTCAAGATACAACATTTCTCCTATAGTCATGGGATTTGTTCTCTTTTCACAATTGCCTTCATTT

M G F V L F S Q L P S F

66 CTTCTTGTCTCTACACTTCTCTTATTCCTAGTAATATCCCACTCTTGCCGTGCCCAAAATTCTCAACAAGACTATTTGGA

L L V S T L L L F L V I S H S C R A Q N S Q Q D Y L D

146 TGCCCATAACACAGCTCGTGCAGATGTAGGTGTAGAACCTTTGACCTGGGACGACCAGGTAGCAGCCTATGCGCAAAATT

A H N T A R A D V G V E P L T W D D Q V A A Y A Q N Y

226 ATGCTTCCCAATTGGCTGCAGATTGTAACCTCGTACATTCTCATGGTCAATACGGCGAAAACCTAGCTGAGGGAAGTGGC

A S Q L A A D C N L V H S H G Q Y G E N L A E G S G

306 GATTTCATGACGGCTGCTAAGGCCGTTGAGATGTGGGTCAATGAGAAACAGTATTATGACCATGACTCAAATACTTGTGC

G(→D)

D F M T A A K A V E M W V N E K Q Y Y D H D S N T C A

386 ACAAGGACAGGTGTGTGGACACTATACTCAGGTGGTTTGGCGTAACTCGGTTCGTGTTGGATGTGCTAGGGTTCAGTGTA

Q G Q V C G H Y T Q V V W R N S V R V G C A R V Q C N

466 ACAATGGAGGATATGTTGTCACTTGCAACTATGATCCTCCAGGTAATTATAGAGGCGAAAGTCCATACTAATTGAAACGA

T(→S)

N G G Y V V T C N Y D P P G N Y R G E S P Y

546 CCTACGTCCATTTCACGTTAATATGTATGGATTGTTCTGCTTGATATCAAGAACTCAAATAATTGCTCTAAAAAGCAACT

T

626 TAAAGTCAAGTATATAGTAATAGTACTATATTTGTAATCCTCTGAAGTGGATCTATAAAAAGACCAAGTGGTCATAATTA

706 AGGGGAAAAATATGAGTTGATGATCAGCTTGATGTATGATCTGATATTATTATGAACACTTTTGTACTCATACGAATCAT

†$A_6$

786 GTGTTGATGGTCTAGCTACTTGCGATATTACGAGCAAAATTCTTAACTACATGCCTTAGGAACAAGCTTACACAGTTCAT

†$A_{48}$

866 ATAATCTACTAGAGGGCCAAAAACATGAAAATTACCAATTTAGATGGTAGGAGGATATTGAAAGTGGAGCAGCTAGTTTT

946 AATAACTGACCGTTAGTCTTAAAATTGACGGTATAAAAATATTTACATAATCAGGTCATTTATAAGGTAATTATAGGTAA

1026 ATA

*FIG. 8*

FIG. 9

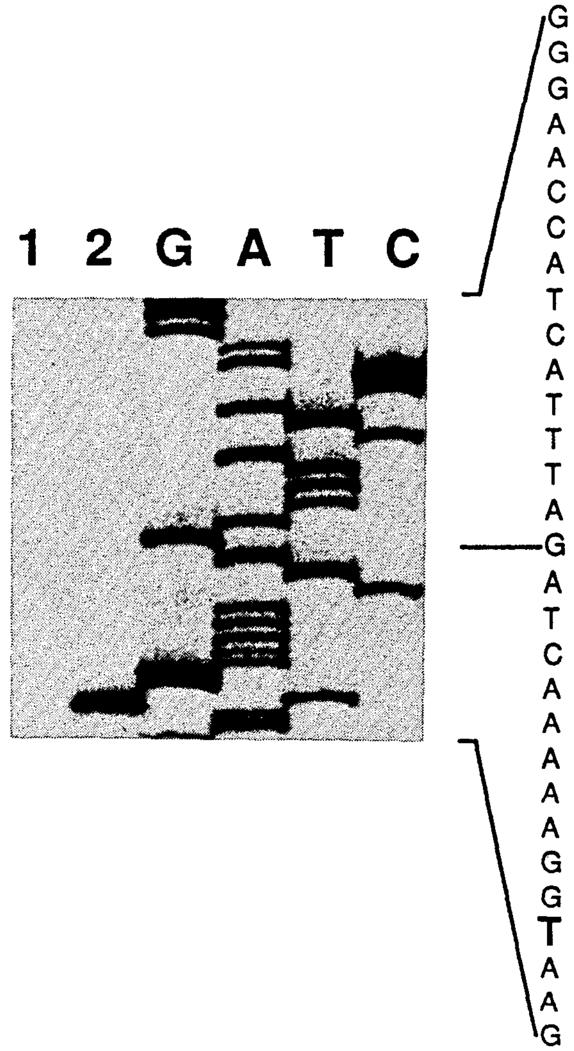

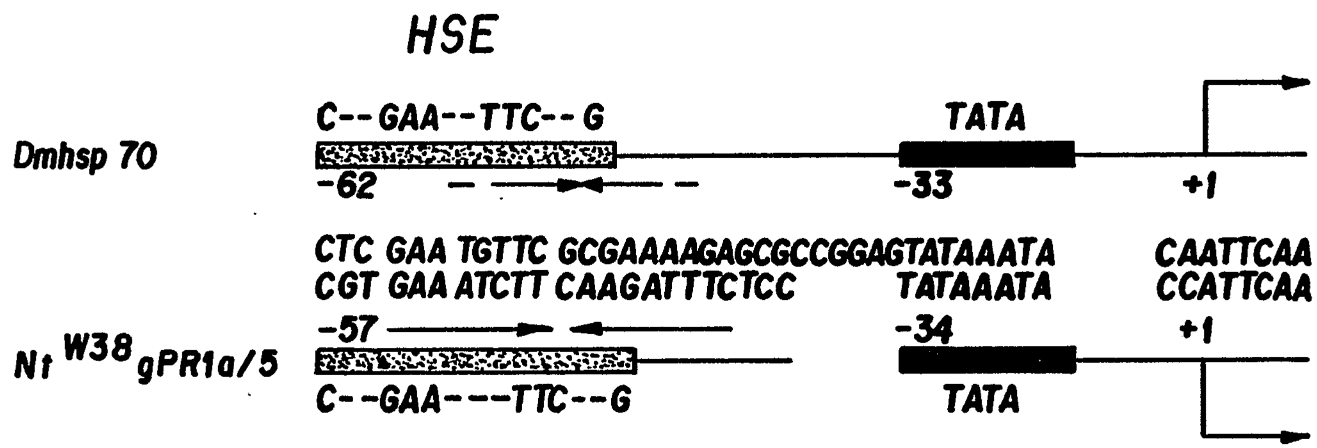
HSE
C--GAA--TTC--G
TATA
Dmhsp 70
-62
-33
+1
CTC GAA TGTTC GCGAAAAGAGCGCCGGAGTATAAATA
CAATTCAA
CGT GAA ATCTT CAAGATTTCTCC
TATAAATA
CCATTCAA
-57
-34
+1
Nt W38 gPR1a/5
C--GAA---TTC--G
TATA

FIG.10

European Patent Office

# EUROPEAN SEARCH REPORT

Application number

EP 88114877.9

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| D,A | THE EMBO JOURNAL, vol. 4, no. 11, 1985, Oxford<br>J. LUCAS et al. "Amino acid sequence of the 'pathogenesis-related' leaf protein p14 from viroid-infected tomato reveals a new type of structurally unfamiliar proteins"<br>pages 2745-2749<br>* Totality *<br>---- | | C 12 N 15/00<br>C 07 H 21/04<br>A 01 H 1/00<br>A 01 N 65/00 |
| | | | TECHNICAL FIELDS SEARCHED (Int. Cl.4) |
| | | | C 12 N<br>C 07 H<br>A 01 N<br>A 01 H |

The present search report has been drawn up for all claims

| Place of search | Date of completion of the search | Examiner |
|---|---|---|
| VIENNA | 12-12-1988 | BÖHM |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document